(19) Europäisches Patentamt
European Patent Office
Office européen des brevets

(11) EP 1 471 181 B1

(12) EUROPEAN PATENT SPECIFICATION

(45) Date of publication and mention
of the grant of the patent:
**23.05.2012 Bulletin 2012/21**

(51) Int Cl.:
***D21C 5/00*** (2006.01)

(21) Application number: **04005659.0**

(22) Date of filing: **10.03.2004**

(54) **Biopulp for non-woody fiber plants and biopulping method thereof**

Biopulpe aus nichtholzartigen Faser-Pflanzen und biologisches Abschlussverfahren dafür

Pâte biologique de plantes fibreuses non-boiseux et procédé de fabrication

(84) Designated Contracting States:
**AT BE BG CH CY CZ DE DK EE ES FI FR GB GR HU IE IT LI LU MC NL PL PT RO SE SI SK TR**

(30) Priority: **25.03.2003 CN 03108335**

(43) Date of publication of application:
**27.10.2004 Bulletin 2004/44**

(73) Proprietor: **Yuen Foong Yu Paper MFG Company, Limited**
**Taipei,**
**Taiwan 411 (TW)**

(72) Inventors:
• **Huang, Jenn-Wen**
**Taiping City**
**Taichung**
**Taiwan 411 (CN)**
• **Peng, Yu-Hsiang**
**Linkou Shiang**
**Taipei**
**Taiwan 244 (CN)**

(74) Representative: **Isarpatent**
**Patent- und Rechtsanwälte**
**Postfach 44 01 51**
**80750 München (DE)**

(56) References cited:
**EP-A2- 0 200 409      WO-A-89/01074**
**JP-A- 7 053 602       JP-A- 7 238 488**
**JP-A- 8 092 893       JP-A- 10 077 595**
**JP-A- 11 061 678      JP-A- 2001 226 898**
**KR-A- 20030 065 916   US-A1- 2002 096 285**

**Description**

The present invention provides a biopulp and a biopulping method thereof, and more particularly to a biopulp for non-woody fiber plants and a biopulping method thereof.

The paper-making industry is an universally traditional industry. The development of paper-making industry is the index of the economy and living standard for a country. The source of paper pulp mostly comes from cutting down the forest. (It needs four metric tons of woods to produce one metric ton of paper pulp. It equals to cut down twenty-three trees.) Because of that, the forest area on the earth is rapidly decreased. The ecological balance problem becomes more and more serious. Furthermore, it needs a great quantity of water and chemicals to wash pulp. However, the waste liquid is discharged from factory in the traditional chemical paper-making process. It also results in environmental pollution. The rivers and oceans are polluted. Nowadays, people in the whole world pay much attention to environmental protection. The corporations in paper-making industry are obliged to spend much money to improve the environmental quality. The production costs are thus raised. Those problems really strike against the paper-making industry.

The annual yield of rice straws is about 2300 thousand metric tons in Taiwan. The organic components of rice straws are almost more than 95%. The organic components include 41.3 % carbon, 0.81 % hydrogen, 20.6 % semicellulose, 24.7 % cellulose and 7.7 % lignin. Conventionally, the handling methods for rice straws include manufacturing them into straw ropes, straw bags, straw mats and cardboards, serving them as covering material for a plot of land, utilizing them as fuel, and mixing them with other material to produce a compost. Also, the rice straw could be directly buried in soil or burned for recyclably using the nutrition. Nowadays, the rice straws are rarely used to be the fuel, feed, straw bags or straw mats because of the expensive costs and the advanced science and technology. Most of the rice straws are locally burned or directly buried in soil, which often result in environmental pollution. On the other hand, since the rice straws are in abundant fiber, it will be very helpful to mitigate the environmental pressure of logging the trees for papermaking if the non-woody fiber plants could be well developed and used. In the past, the fiber production methods by using non-woody fiber plants as original material are generally chemical or semi-chemical methods. However, there exist three difficult problems in the paper-making industry resulting from the chemical production method for pulp. They are described as follows. (1) Large amount of silicates and black liquid with high viscosity produced in the process often result in the serious problems of recycle systems. (2) The deposition of calcium carbonate will be affected by the silicates and thus will lead to the dirt appearance attached on the vapor apparatus. In addition, the piping of evaporator will get undesirable black viscous liquid attached on. Therefore, it needs to stop working for cleaning the apparatus. (3) The unstable status of the steamer and boiling machines will waste the fuel and thus will raise the production costs.

The biotechnology is the key for reorganizing the traditional industry structure. It is a very important direction moved towards the use of the biotechnology for papermaking in the industry. Recently, the advantages of using biotechnology for papermaking are the reduction of production cost, the improvement of pulp quality and the safety maintenance of the working environment, etc. There are many methods and products produced, for example, the removal of the gum or printing ink by using enzymes, paper bleaching by using xylanase or lignin oxidizing enzyme, and the improvement of pulp viscosity by using enzymes (non-woody fiber pulp especially). However, these methods also have the drawbacks of environmental pollution caused by waste liquid and energy consumption and so on. Therefore, it is imperative to seek the assistance of biotechnology for solving and overcoming the drawbacks of papermaking by using chemical methods.

Researchers in many countries of Europe and American attempt to use white-rot fungi, such as *Phanerochaete chrysosporiumand* and *Cereporiopsis subvermispora,* grown on wood slices for removing the lignin of woods and saving the cost and energy of paper making. Although there are some results came from those researches, it takes too much time for the industry to grow the white-rot fungi on woods outdoors.

The published Japanese patent application JP 07 238488 A provides a process for the production of paper comparable to those of wood pulp paper by using a composition composed mainly of a vegetable-originated cellulose as a raw material. This compound material composed of bacterial cellulose and fibers of a cellulose-hemicellulose mixture is formed by inoculating a bacterial strain BPR2001 (FERM BP-4545) belonging to the genus *Acetobacter* to a specific culture medium and culturing the bacterial strain at a prescribed temperature. The culture medium is composed mainly of at least one kind of vegetable-originated cellulose and contains an assimilable carbon source and/or nitrogen source, partially saccharified by cellulase treatment. The compound material is pulped by a paper-making double-disk refiner and paper is formed from the pulp after washing and bleaching.

The published Japanese patent application JP 11 061678 A describes the technical scheme for obtaining a thin layer printing paper having little pinholes by using a fibrous pulp and a bacterial cellulose as principal ingredients. The thin layer printing paper is obtained by using component A (fibrous pulp) and a component B (bacterial cellulose) as e.g. a stationary culture bacterial cellulose or a submerged culture bacterial cellulose as principal ingredients. The papermaking of both components is carried out ordinarily so that the weight ratio of the components A/B may be (99.5/0.5)-(85/15). The obtained paper has up to 20,000/$m^2$ pinholes in the thin layer printing paper and has a basis weight of about 35 g/$m^2$.

US patent application 2002/096285 A1 discloses a surface-sizing agent for providing a recording paper that contains bacterial cellulose and/or plant-originated fine fibrous cellulose (having an average diameter of 10 μm or less), and a cationic polymer. The cationic polymer can be at least partially bonded to the bacterial cellulose and/or the plant-originated fine fibrous cellulose. The cationic polymer can be an acrylic polymer, a vinyl polymer or an allyl polymer each comprising a quaternary amino group.

The main purpose of the present invention is to apply the decomposition ability of the microorganisms for decomposing the organic matters in the papermaking processes of waste straws so as to establish a model of biopulping processes for non-woody fiber plants. The non-woody fiber plants will become an important source of the raw materials of paper pulp. This approach can decrease the consumption of forest reservations and the production of chemical wastes. And then the problems of papermaking are solved.

From the above description, it is known that how to develop a new pulping method with the advantages of low production costs, low or non pollution has become a major problem waited to be solved. In order to overcome the drawbacks in the prior art, a biopulp for non-woody fiber plants and a biopulping method thereof is provided. The particular design in the present invention not only solves the problem described above, but also uses the waste rice straws and a biopulping method to produce paper pulp for paper-making. It does not need to use the chemical or semi-chemical method, and therefore no pollution problems exist. Thus, the invention has the utility for the industry.

Therefore, the present invention provides a biopulp for non-woody fiber plants and a biopulping method thereof which overcomes the disadvantages described above.

It is an object of the present invention to apply the decomposition ability of the microorganisms for decomposing the organic matters in the papermaking processes of waste straws so as to establish a model of biopulping processes of a non-woody fiber plant. The non-woody fiber plants will become an important source of the raw materials of paper pulp. This approach can decrease the consumption of forest reserves and no chemical pollution is produced. And then the problems of papermaking are solved.

It is another object of the present invention to provide a biopulping method for recycling the waste straws and decreasing the cost of papermaking.

In accordance with an aspect of the present invention, a production method for a paper pulp includes steps of a) providing a culture solution, b) adding a fiber plant into the culture solution, c) adding a suspension of a microorganism into the culture solution, characterized in that the microorganism is one selected from a group consisting of a *Bacillus licheniformis* (PMBP-m5), a *Bacillus subtilis* (PMBP-m6) and a *Bacillus amyloliquefaciens* (PMBP-m7), d) fermentatively culturing the culture solution for preparing a pulp solution, e) boiling the pulp solution, f) pulping the pulp solution, and g) screening the pulp solution for isolating a paper pulp from the pulp solution.

Preferably, the fiber plant is a non-woody fiber plant.

Preferably, the fiber plant is pretreated by one selected from a group consisting of a relatively high pressure treatment under a relatively high temperature, a steaming treatment under a relatively high temperature, a boiling treatment under a relatively high temperature, a fumigated treatment and a soaking treatment under a room temperature.

Preferably, the fiber plant is added into the culture solution by a ratio of 4~15%.

Preferably, the microorganism is isolated from one of a non-woody fiber plant and a livestock excrement compost.

Preferably, the microorganism is inoculated at a concentration ranged from 0 to $10^8$ cfu / ml.

Preferably, the microorganism is a Gram positive bacterium.

Preferably, the fermentatively culturing process is proceeded at a temperature ranged from 20 to 50 °C.

Preferably, the fermentatively culturing process is one of a static culture and a shaking culture.

Preferably, the fermentatively culturing process is proceeded over 0~10 days.

Preferably, the step (e) further includes a step of adding 0 ~ 4 % (w/v) CaO into the pulp solution and boiling the pulp solution for 25~40 minutes under 120~150°C.

Preferably, the pulp solution is screened by 18~300 meshes.

In accordance with another aspect of the present invention, a biopulping method for a non-woody fiber plant includes steps of a) providing a culture solution, b) adding a non-woody fiber plant into the culture solution, c) adding a suspension of a microorganism into the culture solution, characterized in that the microorganism is one selected from a group consisting of a *Bacillus licheniformis* (PMBP-m5), a *Bacillus subtilis* (PMBP-m6) and a *Bacillus amyloliquefaciens* (PMBP-m7), d) fermentatively culturing the culture solution for preparing a pulp solution, e) boiling the pulp solution, f) pulping the pulp solution, and g) screening the pulp solution for isolating a paper pulp from the pulp solution.

Preferably, the fiber plant is pretreated by one selected from a group consisting of a relatively high pressure treatment under a relatively high temperature, a steaming treatment under a relatively high temperature, a boiling treatment under a relatively high temperature, a fumigated treatment and a soaking treatment under a room temperature.

Preferably, the inoculation concentration of a microorganism is at a range from 0 to $10^8$ cfu / ml.

Preferably, the step (e) further includes a step of adding 0 ~ 4 % (w/v) CaO into the pulp solution and boiling the pulp solution for 25-40 minutes under 120~150°C.

Preferably, the pulp solution is screened by 18~300 meshes.

In accordance with another aspect of the present invention, a biopulp of a non-woody fiber plant, includes the components of a non-woody fiber plant and a suspension of a microorganism being one selected from a group consisting of a *Bacillus licheniformis* (PMBP-m5), a *Bacillus subtilis* (PMBP-m6) and a *Bacillus amyloliquefaciens* (PMBP-m7). The non-woody fiber plant and the suspension of the microorganism suspension are mixed and fermentatively cultured for preparing the biopulp.

Preferably, the microorganism is a Gram positive bacterium.

Fig. 1 shows the effects of different treatments on the decomposition percentages of rice straw;

Fig. 2 shows the ability of various strains to decompose the rice straw of Japonica rice;

Fig. 3 shows the effects of different inoculation concentrations of PMBIII strain group on the recovery percentages of the rice straw pulp fibers;

Fig. 4 shows the effects of different fermentation culturing periods on the recovery percentages of various straw pulp fibers;

Fig. 5 shows the effects of microorganism fermentation treatment and chemical treatment on the recovery percentages of various straw pulp fibers; and

Fig. 6 shows the flow chart of biopulping method for waste rice straw according to a preferred embodiment of the present invention.

The foregoing and other features and advantages of the present invention will be more clearly understood through the following descriptions with reference to the drawings, wherein:

(A) The effects of various rice straw treatments on the decomposition of rice straws:

The waste rice straws of Japonica rice (*Oryza sativa* L. subsp. japonica) and Indica rice (*Oryza sativa* L. subsp. indica) are provided. The rice straws are sun-dried, cut into small segments at the length of 2-3 cm and pretreated in different ways. For example, the rice straws are pretreated by an autoclave treatment (121°C, 15 1b/in$^2$ for 15 minutes), a steaming treatment under relatively high temperature (100°C for 30 minutes), a boiling treatment under a relatively high temperature (100°C for 30 minutes), a fumigated treatment (Propylene oxide treatment for one day), or a soaking treatment under a room temperature (25~30°C for 30 minutes). The various treatments of rice straws can further affect the pulp recovery efficiency. The detail steps are described as follow. The rice straws are treated by an autoclave treatment (121°C, 15 1b/in$^2$ for 15 minutes), a soaking treatment under a room temperature (25~30°C for 30 minutes), a fumigated treatment (Propylene oxide treatment for one day), a steaming treatment under relatively high temperature (100°C for 30 minutes) respectively. The pretreated rice straws are added into the flasks containing 100 ml sterile water at the amount of 5 % (w/v) and then respectively incubated at 50°C, 200 rpm shaking culture and static culture for a week. Each treatment has duplicate samples. The changes of the rice straws are observed. The decomposition percentage of rice straws is investigated and recorded.

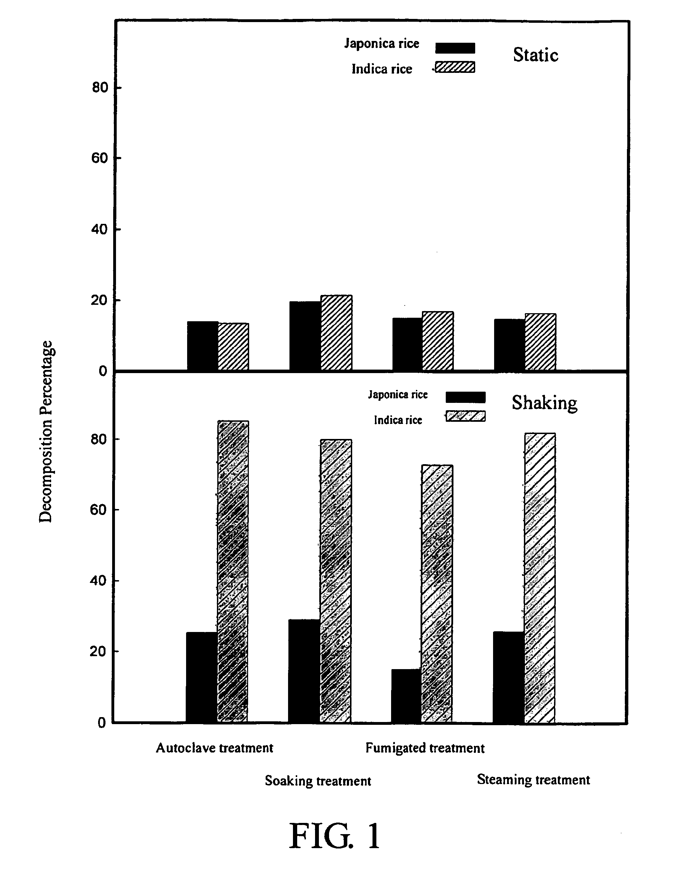

Please refer to Fig. 1, which shows the effects of different treatments on the decomposition percentages of rice straw, which includes an autoclave treatment (121°C, 15 1b/in$^2$ for 15 minutes), a soaking treatment under a room temperature (25~30°C for 30 minutes), a fumigated treatment (Propylene oxide treatment for one day), a steaming treatment under relatively high temperature (100°C for 30 minutes). The decomposition percentage of rice straws is calculated by the following formula.

$$\text{Decomposition \%} = \frac{(\text{Total dry weight of fermentative rice straws} - \text{Dry weight of intact rice straws})}{(\text{Total dry weight of fermentative rice straws})} \times 100$$

The results reveal that the shaking culture is helpful to increase the decomposition of rice straws. After shaking culture, the decomposition percentage of rice straws of Indica rice is obviously higher than that of Japonica rice. The decomposition percentage of the fumigated (Propylene oxide) treatment is quite low in both shaking culture and static culture. It indicates that the microorganisms on the surface of the rice straws are disinfected by the Propylene oxide. Therefore, very few microorganisms are left in the sample treated with propylene oxide. Comparing the effect of soaking treatment under a room temperature with the effect of fumigated treatment, it is proved that the microorganisms are helpful to the decomposition of rice straws. With regard to the steaming treatment under relatively high temperature, the boiling treatment under a relatively high temperature and the soaking treatment under a room temperature, they are all helpful to the decomposition of rice straws. By shaking culture, the aerobic fermentation speeds up the decomposition of the rice straws by the microorganisms.

(B) The selection of bacterial strains having decomposition ability:

The microorganism strains are obtained by the following method according to a preferred embodiment. First, 10 g of the rice straws and 10 g of livestock excrements are prepared and added into 90 ml of sterile water containing agar (0.1 %, w/v). The materials are well mixed and a serious dilution is made. Then, 0.1 ml of $10^3$ X and $10^4$ X diluted solution are uniformly spread on Nutrient Agar plate, pH 8 (NA, purchased Nutrient Agar from Difco company) and Potato Dextrose Agar plate, pH 8 (PDA, purchased Potato Dextrose Agar from Difco company) respectively. Next, the plates are placed in the incubators under 30°C and 50°C for 24 hours and 48 hours respectively. Single colonies grown on plates are picked and isolated for obtaining the microorganism strains. The number of microorganisms isolated from the rice straws and the livestock excrements having the decomposition ability is more than 200 strains. Finally, the microorganisms are identified by the Gram stain. It is found that most of the microorganisms are Gram-positive bacteria.

The isolated microorganisms are further selected by the following steps for selecting the microorganism strains having the decomposition ability for rice straws. (1) 19 strains of the isolated strains, named PMBP-m1, PMBP-m2, PMBP-m3, PMBP-m4, PMBP-m5, PMBP-m6, PMBP-m7, PMBP-O1, PMBP-O2, PMBP-O3, PMBP-O4, PMBP-e1, PMBP-e2, PMBP-e3, PMBP-e4, PMBP-H1, PMBP-H2, PMBP-H3 and PMBP-H4 (as shown in Table 1), are divided into 9 strains groups, including PMBP-I, PMBP-II, PMBP-III, PMBP-IV, PMBP-V, PMBP-VI, PMBP-O, PMBP-E and PMBP-H. Please refer to Table 1, which shows the bacterial strains of different strain groups and the characteristics thereof. (2) The strains groups are cultured with NA plates respectively and then a suspension of microorganism is prepared at the concentration of $10^8$ cfu/ml. (3) 100 ml of solution containing rice straws of Japonica rice (5%, w/v) is prepared. (4) 1 ml of the microorganism suspension is added into the sterile solution prepared in step (3) and then cultured under 50°C and 200 rpm shaking for a week. Each strain is set up in duplicate. (5) The decomposition percentage of rice straws is calculated.

## Table 1

| Isolate / Characteristics | Temp. 50 °C | pH8 | Gram stain (+/-) |
|---|---|---|---|
| PMBP-m1 | ++ | + | + |
| PMBP-m2 | ++ | + | + |
| PMBP-m3 | ++ | + | + |
| PMBP-m4 | ++ | + | + |
| PMBP-m5 | ++ | + | + |
| PMBP-m6 | ++ | + | + |
| PMBP-m7 | ++ | + | + |
| PMBP-O1 | ++ | + | + |
| PMBP-O2 | ++ | + | + |
| PMBP-O3 | ++ | + | + |
| PMBP-O4 | ++ | + | + |
| PMBP-e1 | ++ | + | + |
| PMBP-e2 | ++ | + | + |
| PMBP-e3 | ++ | + | + |
| PMBP-e4 | ++ | + | + |
| PMBP-H1 | ++ | + | + |
| PMBP-H2 | ++ | + | + |
| PMBP-H3 | ++ | + | + |

| PMBP-H4 | ++ | + | + |
|---|---|---|---|

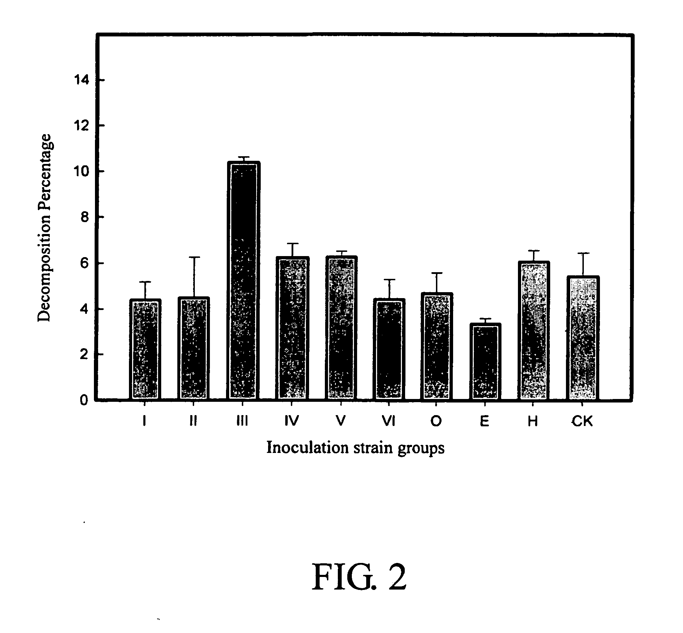

Please refer to Fig. 2, which shows the ability of various strains to decompose the rice straw of Japonica Rice. The Japonica rice straws treated with shaking culturing for a week are classified, dried and weighted. The decomposition percentage of rice straws treated with different microorganisms is calculated by the following formula.

$$\text{Decomposition \%} = \frac{\text{(Total dry weight of fermentative rice straws – Dry weight of intact rice straws)}}{\text{(Total dry weight of fermentative rice straws)}} \times 100$$

As shown in Fig. 2, the PMBIII strain group has the best decomposition ability than the others. The decomposition percentage of rice straws is about 10.38 %. The PMBIII consists of *Bacillus licheniformis* (PMBP-m5), *B. subtilis* (PMBP-m6) and *B. amyloloquefaciens* (PMBP-m7).

(C) The production of biopulp by utilizing bacteria with different inoculation concentrations:

The waste rice straws are the material for producing the biopulp. Different inoculation concentrations of bacteria are added to decompose the rice straws and those of decomposition effects on rice straws are compared. The steps are as follows.

(1) Preparation of culture solution: A LBY culture solution containing 0.25 % lactose, 0.2 % beef extract and 0.05 % Yeast extract is prepared.

(2) Preparation of waste rice straws for testing: The waste rice straws are collected. The cultivated variety of rice is Taichung Sheng No. 10 (Indica rice). The rice straws are sun-dried and cut into small segments at the length of 2-3 cm.

(3) Fermentatively shaking culture: The PMBIII strain group consisting of *Bacillus licheniformis* (PMBP-m5), *B. subtilis* (PMBP-m6) and *B. amyloloquefaciens* (PMBP-m7) is picked and the suspension of PMBIII strain group is prepared. 1000 ml of concave-bottom flasks containing 500 ml LBY culture solutions is prepared. The bacteria suspensions of the PMBIII strain group are added into the culture solution at the concentrations of $1.5 \times 10^4$ cfu/ml (LBY-4 treatment), $1.5 \times 10^6$ cfu/ml (LBY-6 treatment) and $1.5 \times 10^8$ cfu/ml (LBY-8 treatment) respectively. The culture solution without adding any bacteria suspension is the control (LBY-1 treatment). The rice straw segments are added into the culture solutions at the amount of 0.5 % (w/v). And then the culture solutions are fermented in shaking culture under 50°C, 200 rpm for a week. Each concentration of bacteria is set up in four repetitions to prepare a pulp solution.

(4) Boiling of the pulp solution: 1% (w/v) CaO is added into the pulp solution, which is then heated up to 140°C for 30 minutes.

(5) Generation of the pulp solution: The pulp solution is generated by further pulping for 15 minutes.

(6) Filtration of the pulp solution: The pulp solutions are sieved by sieves with 18, 200 and 270 meshes respectively for isolating the incompletely decomposed rice straw pulp from the pulp solutions. The recovery percentages of the rice straw pulp fibers sieved through sieves with different meshes are calculated. The recovered rice straw pulp fibers sieved through 200 meshes are made into the handmade papers. The physical properties of the handmade papers are tested.

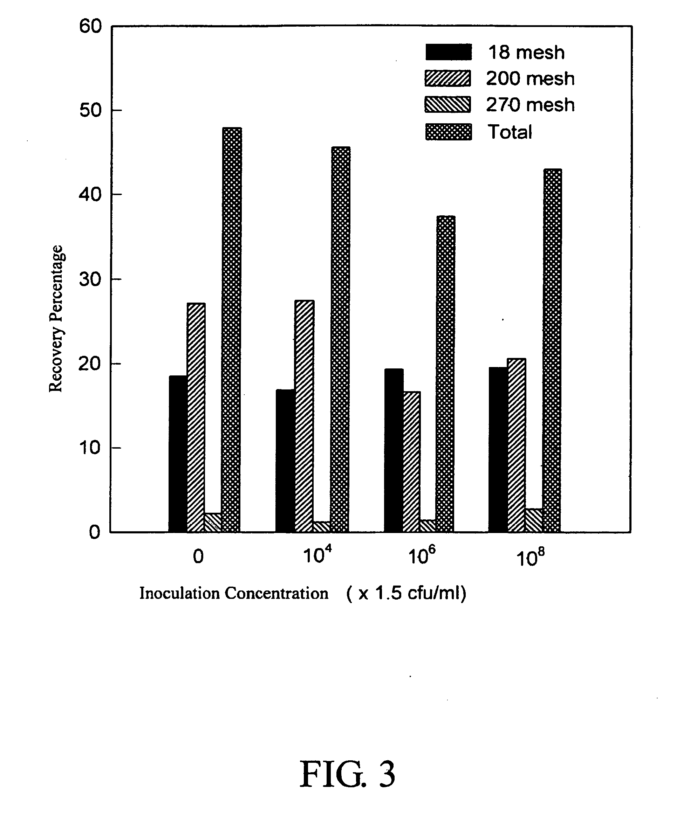

The results are shown in Fig. 3 and Table 2. Fig. 3 shows the effects of different inoculation concentrations of PMBIII strain group on the recovery percentages of the rice straw pulp fibers. The recovery percentages of rice straw pulp fibers are slightly decreasing with increasing inoculation concentrations of PMBIII strain group. High inoculation concentration of PBMIII strain group has no significant effect on the decomposition of rice straws. Please refer to Table 2, which shows the comparisons of physical properties of handmade papers made from the pulp treated with different inoculation concentrations of bacteria. The permeability to gases and the general strength of handmade papers of the LBY-6 treatment (The inoculation concentration is $1.5 \times 10^6$ cfu/ml) is better than the others. The characteristic differences among the papers treated with other inoculation concentration of bacteria are not significant. However, the general strengths of the papers treated with the inoculation of bacteria are all higher than that of the control (LBY-1) which is treated without the inoculation of bacteria.

## Table 2

| Treatment / Test item | LBY-1 C.S.F.:143 ml | LBY-4 C.S.F.:162 ml | LBY-6 C.S.F.:137 ml | LBY-8 C.S.F.:212 ml |
|---|---|---|---|---|
| Basic weight $(g/m^2)$ | 72.4 | 71.0 | 71.7 | 71.4 |
| Thickness (mm) | 0.134 | 0.126 | 0.124 | 0.125 |
| Bulk (ml/g) | 1.85 | 1.77 | 1.73 | 1.75 |
| Breaking length (Km) | 5.74 | 5.69 | 6.24 | 5.99 |
| Tear Index $(mN \cdot m^2/g)$ | 3.74 | 4.14 | 3.50 | 3.90 |
| Burst Index $(Kpa \cdot m^2/g)$ | 2.56 | 2.90 | 3.20 | 3.20 |

| | | | | |
|---|---|---|---|---|
| Cohesion Force (kg-cm) | 2.11 | 2.34 | 2.31 | 2.15 |
| Permeability to Gases (sec/100ml) | 550.8 | 556.5 | 930.2 | 524.0 |
| Surface Strength (A) | 12 | 13 | 13 | 13 |
| Stiffness (g-cm) | 1.52 | 1.36 | 1.36 | 1.42 |
| Opacity (%) | 97.3 | 95.6 | 97.0 | 96.5 |
| Whiteness (%) | 22.3 | 22.2 | 21.7 | 23.1 |
| Ash Content (%) | 11.6 | 11.6 | 11.3 | 11.3 |
| *General Strength | 16.26 | 17.41 | 17.56 | 17.39 |

PS: The treatments of LBY-1, LBY-4, LBY-6 and LBY-8 represent that the inoculation concentration are 0, $10^4$, $10^6$ and $10^8$ cfu/ml respectively.

*General Strength = Breaking length (Km) + Tear index $(mN \cdot m^2/g)$ + Burst Index $(Kpa \cdot m^2/g)$ + [Cohesion Force (kg-cm) x 2]

(D) The effects of different fermentation culturing periods on the production of rice straw pulp fiber:

The length of fermentation culturing time can be various according to a preferred embodiment. First, a LBY liquid medium and the rice straw segments of Indica rice are prepared (The rice straws are sun-dried and cut into small segments at the length of 2-3 cm.). The LBY liquid medium is aliquoted into sterile 1000 ml concaved-bottom flask, 500 ml per flask. The PMBPIII strain group is added into the LBY liquid media at the concentration of $1.5 \times 10^6$ cfu/ml. Then, the rice straw segments are added into the LBY liquid media containing PMBIII strain group at the concentration of 5 % (w/v). And then the mixed solution is cultured in shaking culture at 200 rpm under 50°C for 0, 1, 4, 7 and 10 days respectively. Each treatment is set up in four repetitions. Next, CaO is added into the fermentative culture solution at the concentration of 1 % (w/v) and then the fermentative culture solution is boiled up to 140°C for 30 minutes for preparing the pulp solution. The pulp solution is further pulped for 15 minutes. The pulp solutions are sieved by sieves with 18, 200 and 270 meshes respectively for isolating the incompletely decomposed rice straw pulp fibers from the pulp solutions. The recovery percentages of the rice straw pulp fibers sieved through sieves with different meshes are calculated. The recovered rice straw pulp fibers sieved through 200 meshes are made into the handmade papers. The physical properties of the handmade papers are tested.

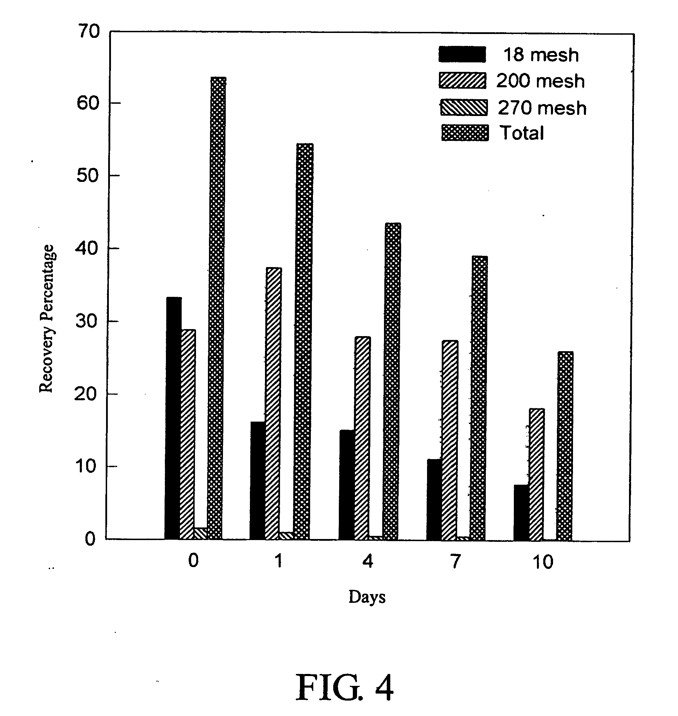

Please refer to Fig. 4 and Table 3. Fig. 4 shows the effects of different fermentation culturing periods on the recovery percentages of various straw pulp fibers. The recovery percentage is decreased as the fermentation culturing period is increased. The pulp fibers recovered from the fibers sieved through 200 meshes, which are fermented for different fermentative periods, are compared. The recovery percentage of 1-day fermentative culture is higher than that of the other periods. Table 3 shows the effects of different fermentation culturing periods on the physical properties of handmade papers made from rice straw pulp fibers. The 4-day fermentative culture has the best gases permeability. And 10-day fermentative culture has the lowest gases permeability. Also, the 4-day fermentative culture has the best general strength.

**Table 3**

| Treatment / Item | LBY-d0 C.S.F.: 209 ml | LBY-d1 C.S.F.: 227 ml | LBY-d4 C.S.F.: 179 ml | LBY-d7 C.S.F.: 138 ml | LBY-d10 C.S.F.: 198 ml |
|---|---|---|---|---|---|
| Basic weight ($g/m^2$) | 72.5 | 71.7 | 70.6 | 72.7 | 73.8 |
| Thickness (mm) | 0.135 | 0.126 | 0.120 | 0.126 | 0.143 |
| Bulk (ml/g) | 1.86 | 1.76 | 1.70 | 1.73 | 1.94 |
| Breaking length (Km) | 3.73 | 4.61 | 5.17 | 4.41 | 3.38 |
| Tear index ($mN \cdot m^2/g$) | 2.49 | 4.05 | 4.00 | 3.56 | 3.89 |
| Burst Index ($Kpa \cdot m^2/g$) | 1.61 | 2.45 | 2.57 | 2.01 | 1.82 |
| Cohesion Force (kg-cm) | 1.76 | 1.75 | 2.04 | 1.69 | 1.69 |
| Permeability to Gases (sec/100ml) | 245.2 | 174.5 | 368.8 | 200.9 | 57.0 |
| Surface Strength (A) | 7 | 9 | 8 | 10 | 7 |
| Stiffness (g-cm) | 1.27 | 1.28 | 1.23 | 1.57 | 1.62 |
| Opacity (%) | 98.7 | 98.4 | 98.2 | 99.1 | 99.3 |
| Whiteness (%) | 18.1 | 22.0 | 22.0 | 24.1 | 22.3 |
| Ash Content (%) | 17.5 | 15.2 | 14.4 | 18.2 | 19.4 |
| *General Strength | 11.35 | 14.61 | 15.82 | 13.36 | 12.47 |

\*General Strength = Breaking length (Km) + Tear index (mN · m$^2$/g) + Burst Index (Kpa · m$^2$/g) + [Cohesion Force (kg-cm) x 2]

(E) The comparison between the biopulping method and the chemical pulping method:

The followings are to compare the differences between biopulping method and chemical pulping method. First, a LBY liquid medium and the rice straw segments of Indica rice are prepared (The rice straws are sun-dried and cut into small segments at the length of 2-3 cm.). The LBY liquid medium is aliquoted into sterile 1000 concaved-bottom flasks, 500 ml per flask. The PMBPIII strain group is added into the LBY liquid media at the concentration of 1.5x10$^6$ cfu/ml. Then, the rice straw segments are added into the LBY liquid media containing PMBIII strain group at the concentration of 5 % (w/v). And then the mixed solution is cultured in shaking culture at 200 rpm under 50°C for 4 days. Each treatment is set up in four repetitions. Next, two treatments are respectively proceeded. First treatment (LBYIII-CaO treatment) is to add CaO into the fermentative culture solution at the concentration of 1 % (w/v) and then boil the fermentative culture solution up to 140°C for 30 minutes for preparing a pulp solution. Second treatment (LBYIII) is to directly boil the fermentative culture solution up to 140°C for 30 minutes for preparing a pulp solution. In addition, the controls are prepared respectively that the rice straw segments are directly mixed with 1 % (w/v) sodium hydroxide solution (NaOH treatment) or 1 % (w/v) CaO solution (CaO treatment). Each treatment is set up in four repetitions. The pulp solutions of all treatments are further pulped for 15 minutes. The pulp solutions are sieved by sieves with 18, 200 and 270 meshes respectively for isolating the incompletely decomposed rice straw pulp fibers from the pulp solutions. The recovery percentages of the rice straw pulp fibers sieved through sieves with different meshes are calculated. The recovered rice straw pulp fibers sieved through 200 meshes are made into the handmade papers. The physical properties of the handmade papers are tested.

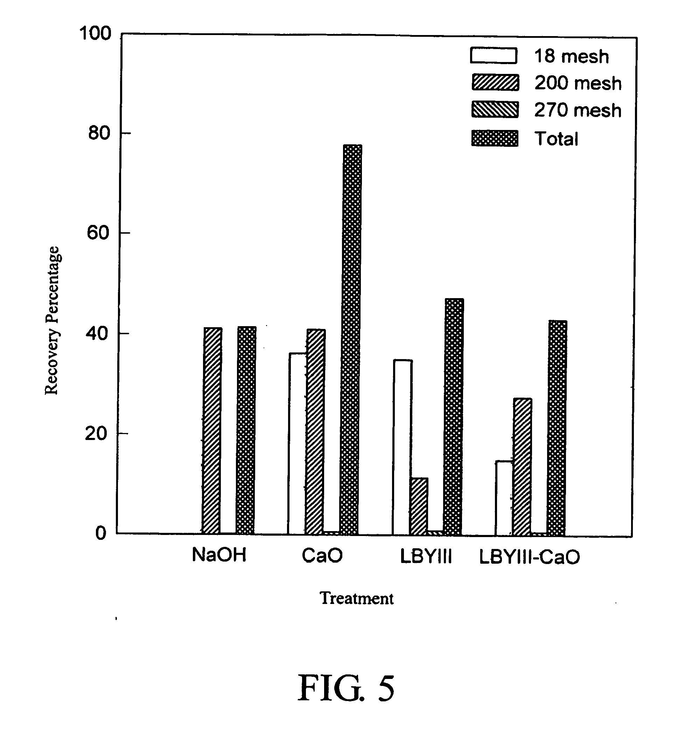

Please refer to Fig. 5, which shows the effects of microorganism fermentation treatment and chemical treatment on the recovery percentages of various rice straw pulp fibers. The total recovery percentage of CaO treatment is the highest. The recovery percentage is 77.79 %. The effect of LBYIII treatment came second, in which the recovery percentage is 47.31 %. The LBYIII-CaO treatment has a recovery percentage of 43.07 %. The recovery percentage of NaOH treatment is 41.45 %, the lowest. When comparing the recovery percentage between the pulp fibers obtained by biopulping method and chemical method, which are recovered from the fibers sieved through 200 meshes, the results of NaOH treatment and the CaO treatment are higher than the other treatments. The result of treatment by microorganism plus CaO (LBYIII-CaO treatment) came second and the result of microorganism treatment (LBYIII treatment) is the lowest. The recovery percentages of the pulp fiber recovered from the fibers sieved through 200 meshes and treated with NaOH, CaO, LBYIII-CaO and LBYIII are 41.21 %, 41.0 %, 27.53 % and 11.45 % respectively.

Please refer to Table 4, which shows the physical properties of handmade papers produced from rice straw pulp fibers which are treated by microorganisms and chemicals. The recovered rice straw pulp fiber sieved through from 200 meshes is made into the handmade papers. The physical properties of the handmade papers are tested. The obtained pulp fibers treated by CaO has the best ionization degree (325 ml), while the obtained pulp fibers treated with LBYIII-CaO has the ionization degree of 267 ml. The handmade paper of LBYIII treatment has the highest gases permeability (302.3 sec/100 ml). The CaO treatment is the lowest (110.3 sec/100 ml). The handmade papers of both NaOH treatment and LBYIII-CaO treatment have the best surface strengths of all treatments (10A and 9A respectively). The handmade paper of the NaOH treatment has the best general strength of all (21.8). The second is the LBYIII-CaO treatment (15.13). The lowest is the LBYIII treatment.

Table 4

| Test item \ Treatment | NaOH C.S.F. : 252 ml | LBYIII C.S.F. : 257 ml | CaO C.S.F. : 325 ml | LBYIII-CaO C.S.F. : 267 ml |
|---|---|---|---|---|
| Basic weight $(g/m^2)$ | 72.8 | 72.9 | 73.3 | 73.4 |
| Thickness (mm) | 0.136 | 0.153 | 0.144 | 0.147 |
| Bulk (ml/g) | 1.87 | 2.10 | 1.96 | 2.00 |
| Breaking length (Km) | 7.21 | 2.87 | 3.36 | 4.89 |
| Tear Index $(mN \cdot m^2/g)$ | 5.99 | 1.28 | 2.61 | 4.21 |
| Burst Index $(Kpa \cdot m^2/g)$ | 4.34 | 0.89 | 1.58 | 2.47 |
| Cohesion Force (kg-cm) | 2.13 | 0.93 | 1.26 | 1.78 |
| Permeability to Gases | 157.7 | 302.3 | 110.3 | 157.3 |

| (sec/100ml) | | | | |
|---|---|---|---|---|
| Surface Strength (A) | 10 | 4 | 7 | 9 |
| Stiffness (g-cm) | 2.20 | 1.57 | 1.38 | 1.55 |
| Opacity (%) | 94.8 | 99.5 | 99.5 | 99.3 |
| Whiteness (%) | 43.5 | 22.7 | 20.4 | 24.9 |
| Ash Content (%) | 4.59 | 13.60 | 20.80 | 16.50 |
| *General Strength | 21.80 | 6.90 | 10.07 | 15.13 |

General Strength = Breaking length + Tear Index + Burst Index + (Cohesion Force x 2)

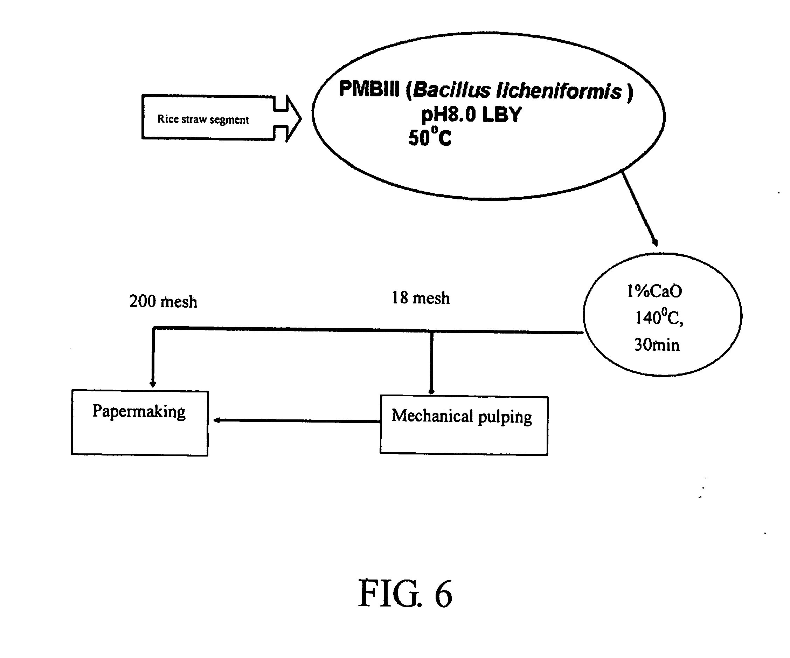

Please refer to Fig. 6, which is the flow chart of biopulping method illustrating the full process of the biopulping method for waste rice straws according to a preferred embodiment of the present invention. First, the rice straw is cut into segments at the length of 2-3 cm. The segments are added into the LBY medium containing $10^6$ (cfu/ml) PMBPIII strain group. The mixed solution is cultured in the shaking culture under 50°C and 200 rpm for four days. The culture solutions are boiled up to 140°C for 30 minutes to prepare pulp solutions. The pulp solutions are further pulped and sieved through sieves for preparing rice straw pulp fibers. And then the papermaking procedure is proceeded.

The bacterial strains described in the present application were deposited with ATCC and have received the following ATCC accession numbers:

*Bacillus licheniformis (PMBP-M5)*
ATCC Number: PTA-5824
Date of Deposit: February 18, 2004

*Bacillus subtilis (PMBP-M6)*
ATCC Number: PTA-5818
Date of Deposit: February 13, 2004

*Bacillus amyloliquefaciens (PMBP-M7)*
ATCC Number: PTA-5819
Date of Deposit: February 13, 2004

**Claims**

1.  A production method for a paper pulp, **characterized in that**:

    (a) providing a culture solution;
    (b) adding a fiber plant into the culture solution;
    (c) adding a suspension of a microorganism into the culture solution, **characterized in that** the microorganism is one selected from a group consisting of a *Bacillus licheniformis* (PMBP-m5), a *Bacillus subtilis* (PMBP-m6) and a *Bacillus amyloliquefaciens* (PMBP-m7);
    (d) fermentatively culturing the culture solution for preparing a pulp solution;
    (e) boiling the pulp solution;
    (f) pulping the pulp solution; and
    (g) screening the pulp solution for isolating a paper pulp from the pulp solution.

2.  The method as claimed in claim 1, **characterized in that** the fiber plant is a non-woody fiber plant.

3.  The method as claimed in claim 1 or 2, **characterized in that** the fiber plant is pretreated by one selected from a group consisting of a relatively high pressure treatment under a relatively high temperature, a steaming treatment under a relatively high temperature, a boiling treatment under a relatively high temperature, a fumigated treatment and a soaking treatment under a room temperature.

4.  The method as claimed in one of the preceding claims, **characterized in that** the fiber plant is added into the culture solution by a ratio of 4~15 %.

5.  The method as claimed in one of the preceding claims, **characterized in that** the microorganism is isolated from one of a non-woody fiber plant and a livestock excrement compost.

6.  The method as claimed in one of the preceding claims, **characterized in that** the microorganism is inoculated at a concentration ranged from 0 to $10^8$ cfu/ml.

7.  The method as claimed in one of the preceding claims, **characterized in that** the microorganism is a Gram positive bacterium.

8.  The method as claimed in one of the preceding claims, **characterized in that** the fermentatively culturing process is proceeded at a temperature ranged from 20 to 50°C.

9.  The method as claimed in one of the preceding claims, **characterized in that** the fermentatively culturing process is one of a static culture and a shaking culture.

10. The method as claim in one of the preceding claims, **characterized in that** the fermentatively culturing process is proceeded over 0~10 days.

11. The method as claimed in one of the preceding claims, **characterized in that** the step (e) further comprises a step of adding 0 - 4 % (w/v) CaO into the pulp solution and boiling the pulp solution for 25-40 minutes under 120~150°C.

12. The method as claim in one of the preceding claims, **characterized in that** the pulp solution is screened by 18~300 meshes.

13. A biopulping method for a non-woody fiber plant, **characterized in that**:

(a) providing a culture solution;
(b) adding a non-woody fiber plant into the culture solution;
(c) adding a suspension of a microorganism into the culture solution, **characterized in that** the microorganism is one selected from a group consisting of a *Bacillus licheniformis* (PMBP-m5), a *Bacillus subtilis* (PMBP-m6) and a *Bacillus amyloliquefaciens* (PMBP-m7);
(d) fermentatively culturing the culture solution for preparing a pulp solution;
(e) boiling the pulp solution;
(f) pulping the pulp solution; and
(g) screening the pulp solution for isolating a paper pulp from the pulp solution.

14. The method as claimed in claim 13, **characterized in that** the fiber plant is pretreated by one selected from a group consisting of a relatively high pressure treatment under a relatively high temperature, a steaming treatment under a relatively high temperature, a boiling treatment under a relatively high temperature, a fumigated treatment and a soaking treatment under a room temperature.

15. The method as claimed in claim 13 or 14, **characterized in that** the inoculation concentration of a microorganism is at a range from 0 to $10^8$ cfu/ml.

16. The method as claimed in one of the claims 13-15, **characterized in that** the step (e) further comprises a step of adding 0 - 4 % (w/v) CaO into the pulp solution and boiling the pulp solution for 25-40 minutes under 120~150°C.

17. The method as claim in one of the claims 13-16, **characterized in that** the pulp solution is screened by 18~300 meshes.

18. A biopulp of a non-woody fiber plant, comprising the components of:

a non-woody fiber plant; and
a suspension of a microorganism being one selected from a group consisting of a *Bacillus licheniformis* (PMBP-m5), a *Bacillus subtilis* (PMBP-m6) and a *Bacillus amyloliquefaciens* (PMBP-m7),
wherein the non-woody fiber plant and the suspension of the microorganism suspension are mixed and fermentatively cultured for preparing the biopulp.

19. The biopulp as claimed in claim 18, **characterized in that** the microorganism is a Gram positive bacterium.

**Patentansprüche**

1. Verfahren zur Herstellung eines Paperbreis, **gekennzeichnet durch**:

(a) Bereitstellen einer Kulturlösung;
(b) Zugeben einer faserhaltigen Pflanze zur Kulturlösung;
(c) Zugeben einer Suspension eines Mikroorganismus zur Kulturlösung, **dadurch gekennzeichnet, dass** der Mirkoorganismus ausgewählt wird aus einer Gruppe, bestehend aus einem *Bacillus licheniformis* (PMBP-m5), einem *Bacillus subtilis* (PMBP-m6) und einem *Bacillus amyloliquefaciens* (PMBP-m7);
(d) fermentatives Kultivieren der Kulturlösung zur Herstellung einer Breilösung;
(e) Kochen der Breilösung;
(f) Aufschließen der Breilösung; und

(g) Sieden der Breilösung, um einen Papierbrei aus der Breilösung zu isolieren.

2. Verfahren nach Anspruch 1, **dadurch gekennzeichnet, dass** die faserhaltige Pflanze eine nicht-holzige Faserpflanze ist.

3. Verfahren nach Anspruch oder 2, **dadurch gekennzeichnet, dass** die faserhaltige Pflanze durch ein Verfahren vorbehandelt wird, ausgewählt aus einer Gruppe, bestehend aus einer Behandlung mit relativ hohem Druck bei einer relativ hohen Temperatur, einer Dampfbehandlung bei einer relativ hohen Temperatur, einer Kochbehandlung bei einer relativ hohen Temperatur, einer Behandlung mit Rauch und einer Behandlung durch Einweichen bei Raumtemperatur.

4. Verfahren nach einem der vorhergehenden Ansprüche, **dadurch gekennzeichnet, dass** die faserhaltige Pflanze zur Kulturlösung in einem Verhältnis von 4 bis 15 % zugegeben wird.

5. Verfahren nach einem der vorhergehenden Ansprüche, **dadurch gekennzeichnet, dass** der Mikroorganismus aus einer nicht-holzigen Faserpflanze und einem Exkrementen-Kompost von Vieh isoliert wird.

6. Verfahren nach einem der vorhergehenden Ansprüche, **dadurch gekennzeichnet, dass** der Mikroorganismus in einer Konzentration im Bereich von o bis $10^8$ cfu/ml eingeimpft wird.

7. Verfahren nach einem der vorhergehenden Ansprüche, **dadurch gekennzeichnet, dass** der Mikroorganismus ein Gram-positives Bakterium ist.

8. Verfahren nach einem der vorhergehenden Ansprüche, **dadurch gekennzeichnet, dass** das Kultivierungsverfahren durch Fermentation bei einer Temperatur im Bereich von 20 bis 50°C ausgeführt wird.

9. Verfahren nach einem der vorgehenden Ansprüche, **dadurch gekennzeichnet, dass** das Kultivierungsverfahren unter Fermentation eine statische Kultur oder eine Schüttelkultur ist.

10. Verfahren nach einem der vorhergehenden Ansprüche, **dadurch gekennzeichnet, dass** das Kultivierungsverfahren durch Fermentation über o bis 10 Tage durchgeführt wird.

11. Verfahren nach einem der vorhergehenden Ansprüche, **dadurch gekennzeichnet, dass** der Schritt (e) weiterhin einen Schritt umfasst, bei welchem o bis 4 % (w/v) CaO zur Breilösung hinzugegeben und die Breilösung 25 bis 40 Minuten lang bei 120 bis 150°C gekocht wird.

12. Verfahren nach einem der vorhergehenden Ansprüche, **dadurch gekennzeichnet, dass** die Breilösung bei einer Siebweite von 18 bis 300 Mesh gesiebt wird.

13. Biologisches Verfahren, um eine nicht-holzige Faserpflanze zu Brei umzuwandeln, **gekennzeichnet durch**:

(a) Bereitstellen einer Kulturlösung;
(b) Hinzugeben einer nicht-holzigen Faserpflanze zur Kulturlösung;
(c) Zugeben einer Suspension eines Mikroorganismus zur Kulturlösung, **dadurch gekennzeichnet, dass** der Mirkoorganismus ausgewählt wird aus einer Gruppe, bestehend aus *Bacillus licheni formis* (PMBP-m5), einem *Bacillus subtilis* (PMBP-m6) und einem *Bacillus* amyloliquefaciens (PMBP-m7);
(d) fermentatives Kultivieren der Kulturlösung zur Herstellung einer Breilösung;
(e) Kochen der Breilösung;
(f) Aufschließen der Breilösung; und
(g) Sieden der Breilösung, um einen Papierbrei aus der Breilösung zu isolieren.

14. Verfahren nach Anspruch 13, **dadurch gekennzeichnet, dass** die faserhaltige Pflanze durch ein Verfahren vorbehandelt wird, ausgewählt aus einer Gruppe, bestehend aus einer Behandlung mit relativ hohem Druck bei einer relativ hohen Temperatur, einer Dampfbehandlung bei einer relativ hohen Temperatur, einer Kochbehandlung bei einer relativ hohen Temperatur, einer Behandlung mit Rauch und einer Behandlung durch Einweichen bei Raumtemperatur.

15. Verfahren nach Anspruch 13 oder 14, **dadurch gekennzeichnet, dass** die Konzentration zum Einimpfen des Mikroorganismus in einem Bereich von 0 bis $10^8$ cfu/ml liegt.

**16.** Verfahren nach einem der Ansprüche 13 bis 15, **dadurch gekennzeichnet, dass** der Schritt (e) weiterhin einen Schritt umfasst, bei welchem 0 bis 4 % (w/v) CaO zur Breilösung hinzugegeben und die Breilösung 25 bis 40 Minuten lang bei 120 bis 150°C gekocht wird.

**17.** Verfahren nach einem der Ansprüche 13 bis 16, **dadurch gekennzeichnet, dass** die Breilösung bei einer Siebweite von 18 bis 300 Mesh gesiebt wird.

**18.** Biologischer Brei einer nicht-holzigen faserenthaltenden Pflanze, umfassend die Bestandteile:

eine nicht-holzige Faserpflanze; und
eine Suspension eines Mikroorganismus, ausgewählt aus einer Gruppe, bestehend aus einem *Bacillus licheniformis* (PMBP-m5), einem Bacillus subtilis (PMBP-m6) und einem Bacillus amyloliquefaciens (PMBP-m7), wobei die nicht-holzige Faserpflanze und die Suspension der Mikroorganismensuspension vermischt und zur Herstellung des biologischen Breis fermentativ kultiviert werden.

**19.** Biologischer Brei in Anspruch 18, **dadurch gekennzeichnet, dass** der Mikroorganismus ein Gram-positives Bakterium ist.

## Revendications

**1.** 1. Procédé pour produire une pâte à papier **caractérisé par** :

(a) la fourniture d'une solution de culture :
(b) l'ajout d'une plante à fibres à la solution de culture ;
(c) l'ajout d'une suspension d'un micro-organisme dans la solution de culture, **caractérisé en ce que** le micro-organisme est choisi parmi un groupe constitué par un *Bacillus licheniformis* (PMBP-m5), un *Bacillus subtilis* (PMBP-m6) et un *Bacillus amyloliquefaciens* (PMBP-m7) ;
(d) la culture par fermentation de la solution de culture pour préparer une solution de pâte ;
(e) l'ébullition de la solution de pâte ;
(f) la réduction en pâte de la solution de pâte ; et
(g) le passage de la solution de pâte au crible pour séparer de la solution de pâte une pâte à papier.

**2.** Procédé selon la revendication 1, **caractérisé en ce que** la plante à fibres est une plante à fibres non boiseuse.

**3.** Procédé selon la revendication 1 ou 2, **caractérisé en ce que** la plante à fibres est préalablement soumise à un traitement choisi parmi un groupe constitué par un traitement à pression relativement élevée à une température relativement élevée, un traitement par étuvage à une température relativement élevée, un traitement par ébullition à une température relativement élevée, un traitement par fumigation et un traitement par trempage à température ambiante.

**4.** Procédé selon l'une des revendications précédentes, **caractérisé en ce que** la plante à fibres est ajoutée dans la solution de culture dans une proportion allant de 4 à 15 %.

**5.** Procédé selon l'une des revendications précédentes, **caractérisé en ce que** le micro-organisme est isolé à partir d'une plante à fibres non boiseuse et d'un compost d'excréments de bétail.

**6.** Procédé selon l'une des revendications précédentes, **caractérisé en ce que** le micro-organisme est inoculé à une concentration allant de 0 à $10^8$ bactéries souches/ml.

**7.** Procédé selon l'une des revendications précédentes, **caractérisé en ce que** le micro-organisme est une bactérie Gram-positive.

**8.** Procédé selon l'une des revendications précédentes, **caractérisé en ce que** l'opération de culture par fermentation est réalisée à une température allant de 20 à 50°C.

**9.** Procédé selon l'une des revendications précédentes, **caractérisé en ce que** l'opération de culture par fermentation est soit une culture statique soit une culture par agitation.

**10.** Procédé selon l'une des revendications précédentes, **caractérisé en ce que** l'opération de culture par fermentation dure de 0 à 10 jours.

**11.** Procédé selon l'une des revendications précédentes, **caractérisé en ce que** l'étape (e) comprend en outre une étape qui consiste à ajouter de 0 à 4 % (M/V) de CaO dans la solution de pâte et à faire bouillir la solution de pâte pendant 25 à 45 minutes à 120-150°C.

**12.** Procédé selon l'une des revendications précédentes, **caractérisé en ce que** la solution de pâte est passée au crible de 18 à 300 mesh.

**13.** Procédé pour réduire en biopulpe une plante à fibres non boiseuse, **caractérisé par** :

(a) la fourniture d'une solution de culture :
(b) l'ajout d'une plante à fibres non boiseuse à la solution de culture ;
(c) l'ajout d'une suspension d'un micro-organisme dans la solution de culture,
**caractérisé en ce que** le micro-organisme est choisi parmi un groupe constitué par un *Bacillus licheniformis* (PMBP-m5), un *Bacillus subtilis* (PMBP-m6) et un *Bacillus amyloliquefaciens* (PMBP-m7) :
(d) la culture par fermentation de la solution de culture pour préparer une solution de pâte ;
(e) l'ébullition de la solution de pâte ;
(f) la réduction en pâte de la solution de pâte ; et
(g) le passage de la solution de pâte au crible pour séparer de la solution de pâte une pâte à papier.

**14.** Procédé selon la revendication 13, **caractérisé en ce que** la plante à fibres est préalablement soumise à un traitement choisi parmi un groupe constitué par un traitement à pression relativement élevée à une température relativement élevée, un traitement par étuvage à une température relativement élevée, un traitement par ébullition à une température relativement élevée, un traitement par fumigation et un traitement par trempage à température ambiante.

**15.** Procédé selon la revendication 13 ou 14, **caractérisé en ce que** le micro-organisme est inoculé à une concentration allant de 0 à $10^8$ bactéries souches/ml.

**16.** Procédé selon l'une des revendications 13 à 15, **caractérisé en ce que** l'étape (e) comprend en outre une étape qui consiste à ajouter de 0 à 4 % (M/V) de CaO dans la solution de pâte et à faire bouillir la solution de pâte de 25 à 40 minutes à 120-150°C.

**17.** Procédé selon l'une des revendications 13 à 16, **caractérisé en ce que** la solution de pâte est passée au crible de 18 à 300 mesh.

**18.** Biopulpe de plante à fibres non boiseuse comprenant les composants suivantes :

- une plante à fibres non boiseuse ; et
- une suspension d'un micro-organisme choisi parmi un groupe constitué par un *Bacillus licheniformis* (PMBP-m5), un *Bacillus subtilis* (PMBP-m6) et un *Bacillus amyloliquefaciens* (PMBP-m7),
- la plante à fibres non boiseuse et la suspension de micro-organisme étant mélangées et cultivées par fermentation pour préparer la biopulpe.

**19.** Biopulpe selon la revendication 18, **caractérisée en ce que** le micro-organisme est une bactérie Gram positive.

FIG. 1

FIG. 2

FIG. 3

FIG. 4

FIG. 5

FIG. 6

**EP 1 471 181 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7238488 A
- JP 11061678 A
- US 2002096285 A1